# EUROPÄISCHE PATENTSCHRIFT

(11) **EP 1 729 673 B1**
(45) Veröffentlichungstag und Bekanntmachung des Hinweises auf die Patenterteilung: **27.05.2009**
(21) Anmeldenummer: 05716414.7
(22) Anmeldetag: 29.03.2005
(51) Int. Cl.: A61F 2/00

(54) **ZERVIKALE ZWISCHENWIRBELPROTHESE**
CERVICAL INTERVERTEBRAL PROSTHESIS
PROTHESE INTERVERTEBRALE CERVICALE

(30) Priorität: 01.04.2004 US 814783
(43) Veröffentlichungstag der Anmeldung: 13.12.2006
(60) Teilanmeldung: 08020107.2
(73) Patentinhaber: Cervitech, Inc., Rockaway, NJ 07866 (US)
(72) Erfinder: LINK, Helmut, D., 22397 Hamburg (DE); MCAFEE, Paul C., Scoliosis and Spine Center, Baltimore, MD 21204 (US)
(74) Vertreter: Glawe, Delfs, Moll
(86) Internationale Anmeldenummer: PCT/EP2005/003252
(87) Internationale Veröffentlichungsnummer: WO 2005/094720

(56) Entgegenhaltungen:
- WO-A-00/66011
- WO-A-02/09626
- WO-A-02/45592
- WO-A-03/075804
- FR-A- 2 822 674
- US-A- 5 246 458
- US-A- 6 120 503
- US-A1- 2003 204 260

## Beschreibung

Zervikale Zwischenwirbelprothesen sind bekannt (EP-A-1 344 508), die zwei mit den benachbarten Wirbelkörperabschlussplatten zu verbindende Deckplatten aufweisen, zwischen denen sich ein Gelenkkern befindet, der den Deckplatten eine Relativbewegung zueinander erlaubt, durch die die gelenkige Beweglichkeit der ersetzten Bandscheibe nachgebildet werden soll. Um die Deckplatten in ihrer vorgesehenen Position zwischen den Wirbelkörpern zu sichern, sind bei der bekannten Prothese mit den Deckplatten Sicherungsplatten verbunden, die sich vom ventralen Rand der Deckplatten lotrecht dazu erstrecken, um auf der Ventralseite des zugehörigen Wirbelkörpers mit diesem verschraubt zu werden. Nicht immer verlaufen die ventralen Flächen der Wirbelkörper, an denen die Sicherungsplatten zuliegen kommen, genau lotrecht zu den Abschlussplatten der Wirbelkörper, an denen die Hauptflächen der Deckflächen zuliegen kommen. Dies verhindert eine vollflächige Anlage der Sicherungsplatten an den Wirbelkörpern und beeinträchtigt die Haltesicherheit der Deckplatten.

Eine zervikale Zwischenwirbelprothese gemäß dem Oberbegriff von Anspruch 1 wird in WO 03/075804 offenbart.

Dieser Mangel wird durch die Merkmale der Erfindung, die in Anspruch 1 angegeben sind, beseitigt. Demnach ist die Sicherungsplatte ein gesonderter Teil, der mit der Deckplatte unstarr verbunden ist. Folglich kann die Sicherungsplatte unabhängig von der Position der Deckplatte die der Lage und Gestalt der Ventralfläche des Wirbelkörpers gemäß Stellung einnehmen. Dies ist auch dann gewährleistet, wenn eine unstarre Verbindung der beiden Teile vorgesehen ist, beispielsweise eine flexible Verbindung oder eine Gelenkverbindung. Eine solche Verbindung kann so ausgeführt sein, daß die Sicherungsplatte nicht nur eine unerwünschte Verschiebung der Deckplatte nach ventral sondern auch nach dorsal verhindert.

Ein Vorteil der Erfindung besteht darin, dass Mikrobewegungen der Deckplatte, die als Folge der normalen Halsbewegung auftreten können, sich nicht auf die Sicherungsplatte übertragen und daher auch nicht zu einer Lockerung von deren Befestigung führen können. Gleichwohl kann es zweckmäßig sein, die Befestigungsschrauben mit einer Sicherungseinrichtung gegen unbeabsichtigtes Lösen auszurüsten.

Es können im Zusammenhang mit einer Prothese zwei Sicherungsplatten vorgesehen sein, die einander gegenüberliegend an den benachbarten Wirbelkörpern befestigt werden, um jeweils die eine bzw. die andere Deckplatte der Prothese festzuhalten. Im allgemeinen genügt es aber, eine Deckplatte durch eine Sicherungsplatte zu sichern, um sämtliche Teile der Prothese hinreichend an einem ventralen Heraustreten aus dem Zwischenwirbelraum zu hindern.

Es versteht sich, dass die Sicherungsplatte eine vorbestimmte Position im Verhältnis zur Deckplatte haben sollte. Sie sollte nämlich hinreichend weit über den Wirbelkörper hinaus vor den Zwischenwirbelraum vorragen, um die Sicherungsfunktion erfüllen zu können. Andererseits sollte sie auch nicht zu weit vorragen, um nicht die Relativbewegung der Wirbelkörper oder der Prothesenteile zueinander bei der Gelenkbewegung zu stören. Zu ihrer Positionierung wird deshalb zweckmäßigerweise ein Instrument verwendet, das als Bohrlehre für die Befestigungsschrauben der Sicherungsplatte ausgebildet ist. Diese Bohrlehre kann mit Einrichtungen versehen sein, die ihr eine vorbestimmte Position gegenüber der implantierten Prothese verleihen. Stattdessen kann sie auch mit einem Prothesenmodell zusammenwirken oder mit diesem fest verbunden sein, das vor der Implantation der Prothese in den Zwischenwirbelraum eingesetzt wird.

Nach einem besonderen Merkmal der Erfindung kann die Sicherungsplatte biologisch abbaubar sein. Das Material ist zu diesem Zweck in solcher Weise gewählt und dimensioniert, dass sie an Ort und Stelle bleibt und ihre Sicherungsfunktion mindestens während derjenigen Zeitspanne ausüben kann, solange die zugehörige Deckplatte der Prothese sich noch nicht endgültig mit dem angrenzenden Knochengewebe verbunden hat. Sobald dies der Fall ist, d.h. wenn das Knochengewebe sich so innig mit der Deckplattenoberfläche verbunden hat, dass mit einer Relativverschiebung unter den obwaltenden Kräften nicht mehr zu rechnen ist, ist die Sicherungsplatte nicht mehr erforderlich. Die Größenordnung dieser Zeitspanne liegt bei einigen Monaten, beispielsweise bei zwei bis sechs Monaten.

Die Erfindung wird im folgenden näher unter Bezugnahme auf die Zeichnungen erläutert, die ein vorteilhaftes Ausführungsbeispiel veranschaulichen. Es zeigen:
- Fig. 1: einen Sagittalschnitt durch die Prothese im im- plantierten Zustand,
- Fig. 2: eine Seitenansicht der Prothese ohne Sicherungs- platte und
- Fig. 3: eine Bohrlehre für die Befestigungsschrauben der Sicherungsplatte.

Zwischen den Wirbeln 1 und 2 der Halswirbelsäule befindet sich ein zwischenwirbelraum, in den die Zwischenwirbelprothese eingesetzt ist, die sich aus einer oberen Deckplatte 3, einer unteren Deckplatte 4 und einem Prothesenkern 5 zusammensetzt. Der Prothesenkern 5 ist durch Profile 6 und einen Riegel 7 an der unteren Deckplatte 4 gehalten. Mit der oberen Deckplatte 3 bildet er ein Gleitflächenpaar 8. Die Deckplatten 3 und 4 weisen eine Sägezahnung 9 auf, durch die sie an den zugehörigen Abschlussplatten der Wirbelkörper 1, 2 gehalten werden. Kurze Flansche 10 mit nach dorsal gewendeten Anschlagflächen 11 verhindern, dass die Deckplatten 3, 4 sich weiter als gewünscht nach dorsal gegenüber den Wirbelkörpern 1, 2 verschieben können. Eine unerwünschte Bewegung nach ventral wird in der Regel durch die Sägezahnform der Profile 9 verhindert. Das gilt mindestens nach dem verstreichen einiger Monate nach der Operation, wenn das Knochengeweb in die Oberfläche der Deckplatten eingewachsen und sich fest damit verbunden hat. Einzelheiten dieser Konstruktion sind beschrieben in der Veröffentlichung WO 03/075804 A1.

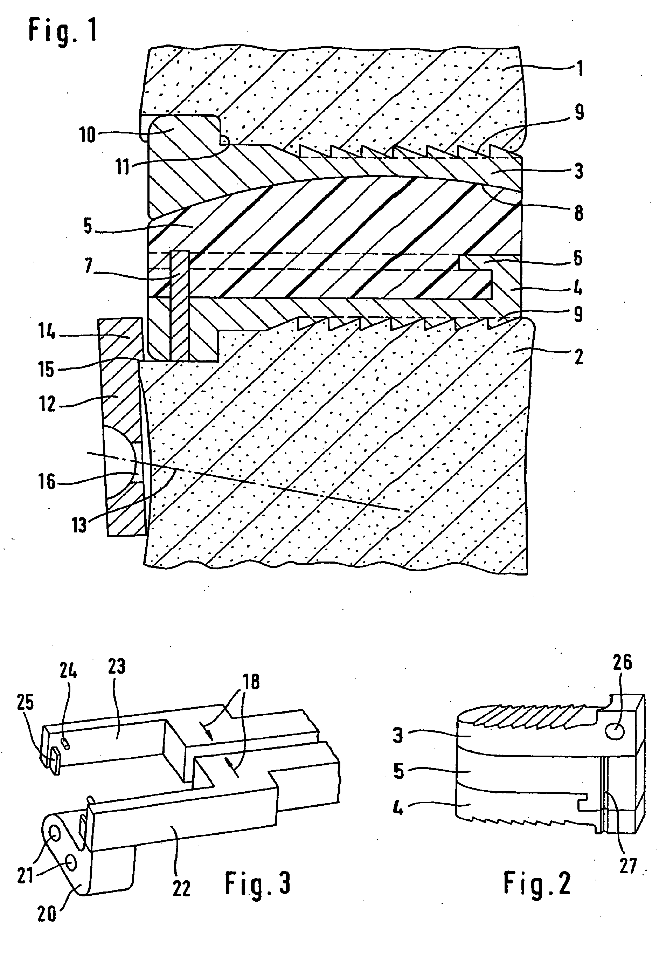

Es gibt Fälle, in denen aufgrund physiologischer Besonderheiten mit der Gefahr einer ventralen Verschiebung der Prothese gerechnet werden muss. Diese Gefahr kann auch zeitweilig bestehen, solange die eben erwähnte Verbindung zwischen den Deckplatten und dem Knochengewebe noch nicht fest ist. In diesen Fällen wird die betreffende Deckplatte mit einer Sicherungsplatte 12 kombiniert, die im Beispiel der Figur 1 an der ventralen Seite des kaudalen Wirbelkörpers 2 mittels Schrauben 13 angebracht ist. Ein als Anschlagteil 14 bezeichneter Teil der Sicherungsplatte 12 ragt über den Wirbelkörper 2 derart hinaus, dass er vor einem Teil der zugeordneten Prothesendeckplatte 4 liegt. Sollte diese eine Tendenz zeigen, sich nach ventral aus dem Zwischenwirbelraum heräuszubewegen, so wird sie gegen den Anschlagteil 14 der Sicherungsplatte 12 stoßen und dadurch an weiterer Bewegung in dieser Richtung gehindert.

Die Sicherungsplatte 12 ist am kaudalen Wirbelkörper 2 gezeigt. Eine Sicherungsplatte könnte aber zusätzlich oder statt dessen auch am kranialen Wirbelkörper 1 angebracht sein.

Die Art der Anbringung ist für die Erfindung ohne Belang. In erster Linie bieten sich Knochenschrauben an, die zweckmäßigerweise mit einer (nicht gezeigten) Lösesicherung versehen werden. Sie können im wesentlichen parallel zur Hauptebene der Prothese im Wirbelkörper eingeschraubt sein. Besonders vorteilhaft ist es, ihnen eine - wie gezeigt - nach dorsal von der Prothese weg gerichtete Neigung zu verleihen.

Auf eine sehr genaue Positionierung der Sicherungsplatte 12 kommt es nicht an. Es genügt, wenn sie an geeigneter Stelle - zweckmäßigerweise über einen wesentlichen Teil der Breite der Prothese - in denjenigen Weg hineinragt, den die Prothese bei einer ungewollten Ventralbewegung nehmen würde. Dafür genügt es, wenn sie 1 oder 2 mm über die Kante 15 des Wirbelkörpers 2 hinausragt. Sie soll nicht mehr als etwa 2,5 bis 3 mm darüber hinaus ragen, um die relative Beugebewegung der Wirbelkörper 1, 2 sowie der Prothesenteile nicht zu behindern.

Um die Positionierung zu erleichtern, kann die Sicherungsplatte 12 mit einer Kante 15 versehen sein, die der mit derselben Bezugsziffer bezeichneten Kante des Wirbelkörpers 2 entspricht und den Anschlagteil 14 der Sicherungsplatte von demjenigen Teil trennt, der an der Frontseite des Wirbelkörpers zu befestigen ist. Wenn der Anschlagteil 14 die gewünschte Höhe von etwa 2 mm aufweist, geht der Chirurg so vor, dass er die Sicherungsplatte an den Wirbelkörper 2 in der Weise anlegt, dass die Kanten 15 der Sicherungsplatte des Wirbelkörpers aneinanderliegen. Danach bohrt er die Löcher für die Aufnahme der Befestigungsschrauben 13, indem er die Schraublöcher 16 in der Sicherungsplatte als Bohrlehren verwendet. Auf diese Weise gelangt er zu einer sicheren Positionierung.

Eine noch sicherere Positionierung gelingt ihm bei Verwendung des in Figur 3 gezeigten Instruments. Eine Bohrlehre 20 mit Bohrungen 21 für die Führung des Bohrers ist an einem zangenartigen Instrument angeordnet, das zwei Schenkel 22, 23 aufweiset, die mit nicht dargestellten Mitteln in Pfeilrichtung 18 einander genähert und in der genäherten Stellung festgehalten werden können. Die Schenkel 22, 23 weisen an ihren einander zugewendeten Flanken Vorsprünge 24, 25 auf, die komplementär zu entsprechenden Ausnehmungen 26, 27 bei der Prothese ausgebildet sind, und zwar handelt es sich in dem dargestellten Beispiel um Zapfen 24, die mit Bohrungen 26 korrespondieren und Klingen 25, die mit Schlitzen 27 korrespondieren. Nachdem die Prothese 3, 4, 5 in den Zwischenwirbelraum eingesetzt wurde, wie Figur 1 es zeigt, wird das Instrument an die Prothese angesetzt und mit Hilfe der Organe 24 bis 27 daran justiert. Die Bohrungen 21 der Bohrlehre 20 befinden sich nun achsgleich an derjenigen Stelle, an der die Bohrungen für die Befestigungsschrauben 13 der Sicherungsplatte vorgesehen werden sollen.

Das in Figur 3 gezeigte Instrument ist speziell für eine kaudal von der Prothese anzuordnenden Sicherungsplatte geeignet. Das liegt an der Anordnung der Organe 24 bis 27. Für die Vorbereitung einer kranial zu befestigenden Sicherungsplatte kann es mit einer entsprechenden Bohrlehre auch an seiner in Figur 3 oben erscheinenden Seite ausgerüstet werden.

Die in Fig. 1 dargestellte Sicherungsplatte kann man sich - von ventral gesehen - etwas rechteckig oder oval mit größerer Abmessung in seitlicher Richtung als in der kaudalkranialen Richtung vorstellen. In einer besonders vorteilhaften Ausführungsform ist sie als kreisrunde Scheibe, ähnlich einem Kleiderknopf, geformt mit einem mittigen Schraubenloch. Der Vorteil dieser Ausführungsform besteht darin, dass bei der Operation lediglich auf eine korrekte Positionierung der Befestigungsschraube, nicht aber auf die Ausrichtung der Sicherungsplatte geachtet zu werden braucht. Die Form der Kreisscheibe hat ferner den Vorteil, dass sie weniger leicht zu Irritation der umliegenden Organe führt als eine rechteckige Platte. Das gilt zumal dann, wenn die Kanten, insbesondere die ventralen Kanten, abgerundet werden.

Die Sicherungsplatte kann aus Metall oder einem hinreichend widerstandsfähigen Kunststoff bestehen. Wenn die mit ihr zusammenwirkende Deckplatte aus Metall besteht, wird bevorzugt ein Kunststoff gewählt oder eine Kunststoffzwischenlage, die die nach dorsal gewendete Fläche des Anschlagteils 14 bildet.

Wenn die Sicherungsfunktion der Sicherungsplatte nur zeitweilig erforderlich ist, beispielsweise bis die Deckplatten der Prothese hinreichend mit dem angrenzenden Knochengewebe verwachsen sind, kann die Sicherungsplatte samt ihrer Befestigung, beispielsweise der Schraube 13, aus biologisch abbaubarem Material bestehen. Solches Material ist bekannt und bedarf daher hier keiner Erläuterung. Es wird vom Organismus angegriffen und irgendwie aufgelöst. Die Zeitspanne, innerhalb der dies geschieht, lässt sich durch geeignete Materialwahl beeinflussen. Sie wird so gewählt, dass die Sicherungsplatte und ihre Befestigung hinreichende Sicherungskraft aufbringen kann, solange dies erforderlich ist, beispielsweise für eine Zeitdauer von vier Monaten nach der Operation.

## Patentansprüche

1. Zervikale, gelenkige Zwischenwirbelprothese mit mindestens einer mit einem der beiden benachbarten Wirbelkörper (1, 2) zu verbindenden Deckplatte (3, 4), die einen diese sichernden Flansch (10) mit einer nach dorsal gewendeten Anschlagfläche (11) aufweist, **dadurch gekennzeichnet, dass** zusätzlich eine an dem Wirbelkörper (1, 2) zu befestigende Sicherungsplatte (12) vorgesehen ist, die ein gesonderter, unstar mit der Deckplatte (3, 4) verbundener Teil ist.

2. Zwischenwirbelprothese nach Anspruch 1, **dadurch gekennzeichnet, dass** die Sicherungsplatte - ggf. mit der dafür vorgesehenen Befestigung - biologisch abbaubar ist.

3. Zwischenwirbelprothese nach einem der Ansprüche 1 bis 2, **dadurch gekennzeichnet, dass** die Sicherungsplatte eine Kreisscheibe ist.

## Claims

1. Cervical, jointed intervertebral prosthesis with at least one cover plate (3, 4) to be connected to one of the two adjacent vertebral bodies (1, 2), which has a flange (10) which secures this cover plate (3, 4) and has a limit stop surface (11) facing in the dorsal direction, **characterized in that** a securing plate (12) is additionally provided which is to be fastened to the vertebral body (1, 2) and which is a separate part not rigidly connected to the cover plate (3, 4).

2. Intervertebral prosthesis according to Claim 1, **characterized in that** the securing plate, if appropriate with the fastening means provided for it, is biodegradable.

3. Intervertebral prosthesis according to one of Claims 1 to 2, **characterized in that** the securing plate is a circular disk.

## Revendications

1. Prothèse intervertébrale cervicale articulée avec au moins une plaque de recouvrement (3, 4) à assembler à un des deux corps vertébraux voisins (1, 2), laquelle présente une bride (10) fixant celle-ci avec une face de butée (11) tournée en direction dorsale, **caractérisée en ce qu'**il est en plus prévu une plaque de fixation (12) à fixer au corps vertébral (1, 2), qui est une pièce séparée, reliée de façon non rigide à la plaque de recouvrement (3, 4).

2. Prothèse intervertébrale selon la revendication 1, **caractérisée en ce que** la plaque de fixation - le cas échéant avec la fixation prévue à cet effet - est biologiquement dégradable.

3. Prothèse intervertébrale selon l'une des revendications 1 à 2, **caractérisée en ce que** la plaque de fixation est un disque circulaire.
